# FASCICULE DE BREVET EUROPEEN

(11) **EP 3 259 247 B1**
(45) Date de publication et mention de la délivrance du brevet: **23.10.2019**
(21) Numéro de dépôt: 16705128.3
(22) Date de dépôt: 17.02.2016
(51) Int. Cl.: C07C 67/08, C07C 1/213

(54) **PROCÉDÉ DE PRODUCTION DE 1,3-BUTADIÈNE À PARTIR DE 1,4-BUTANEDIOL**
VERFAHREN ZUR HERSTELLUNG VON 1,3-BUTADIEN AUS 1,4-BUTANDIOL
PROCESS FOR PRODUCING 1,3 BUTADIENE FROM 1,4 BUTANEDIOL

(30) Priorité: 18.02.2015 FR 1551353
(43) Date de publication de la demande: 27.12.2017
(73) Titulaire: IFP Energies nouvelles, 92500 Rueil-Malmaison (FR); Compagnie Générale des Etablissements Michelin, 63000 Clermont-Ferrand (FR)
(72) Inventeur: RICHARD, Romain, 38070 St Quentin Fallavier (FR); JACQUIN, Marc, 69002 Lyon (FR); DORATO, Margarita, 63040 Clermont-Ferrand Cedex 9 (FR); PACHECO, Nuno, 63040 Clermont-Ferrand Cedex 9 (FR); RANNOUX, Claire, 63040 Clermont-Ferrand Cedex 9 (FR)
(74) Mandataire: IFP Energies nouvelles
(86) Numéro de dépôt international: PCT/EP2016/053301
(87) Numéro de publication internationale: WO 2016/131846

(56) Documents cités:
- US-A- 2 345 113
- HE, PING ET AL: "Acetylation of alcohols and phenols using continuous-flow, tungstosilicic acid-supported, monolith microreactors with scale-up capability", JOURNAL OF FLOW CHEMISTRY, vol. 2, 2012, pages 47-51, XP002751790, ISSN: 2062-249X, DOI: 10.1556/JFC-D-12-00002

## Description

### DOMAINE TECHNIQUE DE L'INVENTION

L'invention concerne la production de 1,3-butadiène à partir de 1,4-butanediol.

### ART ANTÉRIEUR

Aujourd'hui, 95% de la production de 1,3-butadiène est assurée par le vapocraquage d'hydrocarbures et l'extraction subséquente des dioléfines au sein d'une coupe de distillation C₄ par des procédés de distillation extractive.

L'évolution du prix des matières premières conduit à opérer les unités de vapocraquage avec des charges de plus en plus légères, car moins coûteuses, entrainant la diminution de production de coupe C₄, et par conséquent de 1,3-butadiène. Des procédés alternatifs de production de 1,3-butadiène doivent donc être trouvés.

Un procédé de production de 1,3-butadiène à partir de 1,4-butanediol, opéré en Allemagne au début des années 1940, est décrit dans BIOS Final Report n°1060, German Acetylene Chemical Industry. Le procédé met en oeuvre un catalyseur de déshydratation qui produit du 1,3-butadiène à partir d'une charge 1,4-butanediol. Cependant, ce procédé produit très majoritairement du TetraHydroFurane (THF) et très minoritairement du 1,3-butadiène. Le THF produit est recyclé vers l'unité catalytique pour produire davantage de 1,3-butadiène. Le recyclage du THF vers l'unité catalytique est tel que ce procédé est peu attractif d'un point de vue économique. Le document US2345113 décrit également un procédé de production de 1,3-butadiène à partir de 1,4-butanediol.

Un autre procédé de production de 1,3-butadiène à partir de 2,3-butanediol, opéré à l'échelle pilote dans les années 1945 aux USA, est décrit dans les brevets FR 859902, US 2383205, US 2372221, et dans Industrial & Engineering Chemistry, 37 (9), 1945, p.865 à 908. Ce procédé est constitué de deux étapes principales :
- l'estérification du 2,3-butanediol par un acide carboxylique pour former le diester correspondant ;
- la pyrolyse du diester pour produire du 1,3-butadiène et de l'acide carboxylique, ce dernier étant recyclé à l'étape d'estérification.

Ce procédé a été mis au point car la déshydratation directe du 2,3-butanediol conduit à la formation très majoritaire de méthyl-éthyl-cétone (MEK) et que, contrairement au THF, la MEK ne peut pas être déshydratée en 1,3-butadiène. Ce procédé est particulièrement intéressant car l'étape de pyrolyse du diester de 2,3-butanediol peut être réalisée avec de très bons rendements (typiquement plus de 80% mol.), et le 1,3-butadiène obtenu est de grande pureté (typiquement plus de 99% pds.), ce qui est crucial pour son utilisation dans diverses applications (chimie fine, élastomère).

Néanmoins, ce procédé présente certains inconvénients. On peut citer le fait que le la production de butadiène à partir de 2,3-butanediol en passant par l'intermédiaire diester de 2,3-butanediol, produit de la MEK en quantité significative. Cette MEK est produite aussi bien dans l'étape d'estérification du diol, que dans l'étape de pyrolyse du diester. Outre la perte de rendement matière, la production massive de cette impureté pose des problèmes dans les étapes de séparation (homogénéisation des colonnes de distillation azéotropiques hétérogènes), et nécessite l'ajout de colonnes à distiller supplémentaires dédiées à son élimination.

Par ailleurs, on peut citer le fait que le liquide obtenu à l'étape de pyrolyse du diester de 2,3-butanediol est un mélange complexe, qu'il est difficile de séparer pour obtenir :
- l'acide carboxylique que l'on souhaite recycler à l'étape d'estérification,
- les impuretés que l'on souhaite éliminer du procédé,
- les intermédiaires de pyrolyse et le diester de 2,3-butanediol non converti que l'on souhaite recycler à l'étape de pyrolyse.

Dans le procédé de production de 1,3-butadiène à partir de 2,3-butanediol opéré dans les années 1945 aux USA, de l'eau était ajoutée au liquide pyrolytique, afin de pouvoir éliminer par distillation azéotropique hétérogène les impuretés, les intermédiaires de pyrolyse et le diester de 2,3-butanediol de l'acide acétique. Ainsi, l'acide acétique récupéré après cette distillation était riche en eau, et devait être séché au sein d'une autre distillation azéotropique hétérogène avant de pouvoir être recyclé à l'étape d'estérification. L'opération de recyclage de l'acide carboxylique étaient donc délicate, et représentait une part significative des coûts opératoires et d'investissement du procédé.

La présente invention permet de remédier à un ou plusieurs problèmes de l'art antérieur. En effet, la demanderesse a découvert que l'utilisation d'une charge 1,4-butanediol permettait une amélioration globale des performances aussi bien au niveau des différentes étapes du procédé (estérifications, pyrolyse, séparations) qu'au niveau global avec des recyclages simplifiés, une gestion des impuretés améliorée et des pertes plus faibles.

### OBJET ET INTÉRÊT DE L'INVENTION

L'invention concerne un procédé de conversion alimenté par une charge 1,4-butanediol, ledit procédé comprenant au moins :
a) une étape d'estérification comprenant une section de réaction alimentée par la charge 1,4-butendiol et un flux comprenant majoritairement de l'acide carboxylique, comprenant également au moins une section de séparation séparant l'effluent de la section de réaction en au moins un effluent diester de 1,4-butanediol, un effluent eau, et un effluent acide carboxylique, ladite section de réaction étant mise en oeuvre en présence d'un catalyseur acide, à une pression comprise entre 0,01 et 1,0 MPa et à un débit molaire de diol alimentant ladite section sur le nombre de mole de catalyseur dans ladite section compris entre 0,05 et 25 h⁻¹ ;
b) une étape de pyrolyse comprenant un réacteur de pyrolyse alimenté au moins par ledit effluent diester de 1,4-butanediol issu de l'étape a) d'estérification, opéré à une température comprise entre 500 et 650°C de manière à produire un effluent de pyrolyse, ladite étape de pyrolyse comprenant également au moins une section de séparation dans laquelle ledit effluent de pyrolyse est refroidi à une température inférieure à 100°C de manière à produire au moins un effluent de pyrolyse liquide et un effluent de pyrolyse vapeur, ledit effluent de pyrolyse vapeur étant comprimé et/ou refroidi dans ladite section de séparation de manière à condenser le 1,3-butadiène en un effluent 1,3-butadiène.

Un avantage de l'invention est d'améliorer la sélectivité et la conversion, aux étapes d'estérification et de pyrolyse, en utilisant une charge 1,4-butanediol en lieu et place d'une charge 2,3-butanediol. L'amélioration de la conversion permet de réduire les investissements, en réduisant la taille des équipements pour une même quantité de 1,3-butadiène produite. L'amélioration de la sélectivité a un effet direct sur la rentabilité du procédé en consommant moins de charge butanediol pour une même quantité de 1,3-butadiène produite. L'amélioration de la sélectivité a aussi un effet indirect en produisant moins d'impuretés, et donc réduisant les coûts associés à l'élimination de ces impuretés. Une autre avantage de l'invention est de faciliter le recyclage vers l'étape d'estérification de l'acide carboxylique libéré à l'étape de pyrolyse, en produisant un liquide pyrolytique dont les différents constituants peuvent être séparés par simple distillation, réduisant ainsi les coûts d'opération et d'investissement.

Un autre aspect de l'invention est d'accroître le rendement global du procédé en valorisant le THF produit lors de l'utilisation d'une charge 1,4-butanediol.

La demanderesse a découvert que la pyrolyse du diester de 1,4-butanediol pouvait être réalisée dans les mêmes conditions que celle du diester de 2,3-butanediol avec une meilleure conversion et une meilleure sélectivité, tout en produisant non seulement moins d'intermédiaires de pyrolyse, mais de plus des intermédiaires de pyrolyses valorisables sous forme de 1,3-butadiène après conversion, permettant d'accroître le rendement global du procédé avec un enchainement d'étapes, en particulier d'étapes de séparation, plus simple par rapport aux procédés selon l'art antérieur.

### DESCRIPTION DÉTAILLÉE DE L'INVENTION

### Charge

Conformément à l'invention, le procédé est alimenté par une charge 1,4-butanediol comprenant au moins 90% pds de 1,4-butanediol. Ladite charge 1,4-butanediol peut également comprendre de l'eau, avantageusement au plus 10% pds d'eau. Ladite charge 1,4-butanediol peut provenir d'un procédé de fermentation de sucres ou de gaz de synthèse. Ladite charge 1,4-butanediol peut provenir d'un procédé de synthèse du 1,4-butanediol à partir d'acétylène et de formaldéhyde.

### Étape a) d'estérification du 1,4-butanediol

Le procédé de conversion selon l'invention comprend une étape d'estérification a) comprenant une section de réaction alimentée par la charge 1,4-butendiol, un flux comprenant majoritairement de l'acide carboxylique, lequel comprend avantageusement l'effluent acide carboxylique issu de la section de séparation de l'étape a), éventuellement l'effluent de pyrolyse liquide ou le flux d'acide carboxylique issus de l'étape b) et éventuellement un appoint d'acide carboxylique ; ladite section de réaction produisant le diester correspondant et de l'eau, l'étape a) du procédé selon l'invention comprenant également au moins une section de séparation séparant l'effluent de la section de réaction en au moins un effluent diester de 1,4-butanediol, un effluent eau, et un effluent acide carboxylique, ce dernier pouvant avantageusement être recyclé dans l'alimentation en acide carboxylique de la section de réaction.

Par flux comprenant majoritairement de l'acide carboxylique, on entend que ce flux comprend plus de 50% poids d'acide carboxylique.

Dans un arrangement particulier du procédé selon l'invention, ledit flux comprenant majoritairement de l'acide carboxylique est constitué dudit effluent acide carboxylique et dudit effluent de pyrolyse liquide issu de l'étape b).

De manière préférée, l'acide carboxylique utilisé est choisi parmi l'acide formique, l'acide acétique, l'acide propanoïque, l'acide butanoïque ou l'acide benzoïque. De manière très préférée, l'acide carboxylique utilisé est l'acide acétique.

Ladite section de réaction peut être réalisée par tout mode bien connu de l'homme du métier. Elle est mise en oeuvre en présence d'un catalyseur acide, qui peut être homogène ou hétérogène. La MMH dans la section de réaction, c'est-à-dire le débit molaire de diol alimentant ladite section sur le nombre de mole de catalyseur dans ladite section, est comprise entre 0,05 et 25 h⁻¹, préférentiellement entre 0,15 et 20 h⁻¹. Elle est opérée à une pression comprise entre 0,01 et 1,0 MPa, de manière préférée entre 0,05 et 0,2 MPa, et de manière très préférée entre 0,08 et 0,12 MPa.

Ledit catalyseur acide hétérogène est, de manière non limitative, une résine acide échangeuse d'ions (de type Amberlyst, Amberlite, Dowex, et en particulier une Amberlyst 35, une Amberlyst 36 ou une Amberlyst 70), un oxyde mixte (ZrO₂, SnO) ou une zéolithe acide (H-MOR, H-MFI, H-FAU et H-BEA).

De manière préférée, ledit catalyseur acide hétérogène est stable à une température supérieure à 130°C, de manière préférée supérieure à 150°C, de manière très préférée supérieure à 170°C.

Les catalyseurs acide utilisés pour catalyser la réaction d'estérification activent aussi les réactions de déshydratation, notamment à des températures élevées telles que celles citées ci-dessus. Dans le procédé selon l'invention où une charge 1,4-butanediol est utilisée, du THF est ainsi produit par déshydratation.

Conformément à l'invention, ladite étape a) comprend au moins une section de séparation séparant l'effluent de ladite section de réaction en au moins un effluent diester de 1,4-butanediol, un effluent eau, et un effluent acide carboxylique.

De manière préférée, la section de réaction de l'étape d'estérification est mise en oeuvre dans une colonne de distillation réactive, dans laquelle la charge 1,4-butanediol est introduite dans la partie supérieure de la colonne, et l'acide carboxylique est introduit dans la partie inférieure de la colonne. Le rapport des débits molaires de 1,4-butanediol et d'acide carboxylique est compris entre 2 et 6, de manière préférée entre 2 et 4, de manière très préférée entre 2 et 3,5.

Ladite colonne de distillation réactive produit en tête un distillat constitué principalement d'eau produite par la réaction d'estérification et d'acide carboxylique introduit en excès ; et en fond un résidu constitué principalement de diester de 1,4-butanediol et éventuellement d'acide carboxylique. Cette étape de conversion est telle que la conversion de 1,4-butanediol en diester de 1,4-butanediol est supérieure à 95% mol., de préférence supérieure à 99% mol..

Dans l'arrangement préféré où l'étape d'estérification comprend une colonne de distillation réactive, si le catalyseur acide est homogène, il est introduit dans la partie supérieure de la colonne avec la charge 1,4-butanediol ; si le catalyseur est hétérogène, il est maintenu dans la colonne à distiller réactive à l'aide d'un dispositif bien connu de l'homme du métier.

La température de la colonne à distiller est comprise entre la température d'ébullition de l'eau produite en tête, et celle du diester de 1,4-butanediol produit en fond. Dans le cas où l'acide carboxylique utilisé est l'acide acétique, la température entre la tête et le fond de la colonne à distiller varie typiquement entre 100 et 230°C.

Les impuretés issues de la déshydratation de 1,4-butanediol sont éliminées en tête de la colonne de distillation réactive, avec l'eau produite et l'acide carboxylique introduit en excès.

Dans l'arrangement préféré où l'étape d'estérification comprend une colonne de distillation réactive, le distillat de ladite colonne de distillation réactive comprend l'eau produite par la réaction d'estérification, l'acide carboxylique introduit en excès, et le sous-produit issu de la déshydratation de la charge 1,4-butanediol (THF). Ce distillat est séparé en un effluent eau exempt d'acide carboxylique, et qui est éliminé du procédé, et un effluent acide carboxylique exempt d'eau qui est avantageusement recyclé vers la section de réaction. Cette séparation peut être réalisée par tout mode bien connu de l'homme du métier. De manière préférée, dans le cas où l'acide acétique est utilisé pour réaliser l'estérification du 1,4-butanediol, cette séparation est réalisée par distillation azéotropique hétérogène, en utilisant un entraineur. De façon non limitative, cet entraineur peut-être l'isopropyl acétate, le diethtyl éther ou encore l'éthyl tertiobutyl éther.

Un effluent THF comprenant le THF produit est avantageusement séparé de l'effluent de ladite section de réaction, avantageusement dudit distillat de ladite colonne de distillation réactive, par tout moyen connu de l'homme du métier, tel que distillation, extraction liquide-liquide, adsorption, pour éviter son accumulation dans ladite section de séparation.

La demanderesse a découvert que la cinétique et la thermodynamique d'estérification du 1,4-butanediol étaient plus favorable que celle du 2,3-butanediol. L'étape d'estérification a) de la charge 1,4-butanediol selon l'invention permet donc de réduire le temps de séjour dans ladite section réactionnelle, avantageusement dans ladite colonne de distillation réactive, ainsi que la quantité d'acide carboxylique introduit, en comparaison à l'étape d'estérification de l'art antérieur mettant en oeuvre une charge 2,3-butanediol. Cette différence de réactivité entre les deux butanediols et la nature des sous-produits générés conduisent, dans le cadre du procédé selon l'invention, à des équipements de plus faible taille et à une gestion simplifiée des recyclages.

### Étape b) de pyrolyse du diester de 1,4-butendiol

Le procédé de conversion selon l'invention comprend une étape de pyrolyse comprenant un réacteur de pyrolyse alimenté au moins par ledit effluent diester de 1,4-butanediol issu de l'étape a) d'estérification, opéré à une température comprise entre 500 et 650°C de manière à produire un effluent de pyrolyse, ladite étape de pyrolyse comprenant également au moins une section de séparation dans laquelle ledit effluent de pyrolyse est refroidi à une température inférieure à 100°C de manière à produire au moins un effluent de pyrolyse liquide, qui est avantageusement recyclé à l'étape a) d'estérification et un effluent de pyrolyse vapeur.

La réaction de pyrolyse peut être mise en oeuvre en présence ou non d'un catalyseur.

La réaction de pyrolyse transforme principalement une mole de diester de 1,4-butanediol en une mole de 1,3-butadiène et libère ainsi deux moles d'acide carboxylique. Par principalement, on entend que plus de 70%mol du diester de 1,4-butanediol est converti en 1,3-butadiène. Préférentiellement, plus de 80%mol du diester de 1,4-butanediol est converti en 1,3-butadiène. Ledit réacteur de pyrolyse, appelé aussi four de pyrolyse, est opéré à une température avantageusement comprise entre 400 et 700°C, préférentiellement entre 500 et 650°C, de préférence entre 550 et 600°C, de manière préférée entre 575 et 585°C. Le temps de contact optimal au sein du four de pyrolyse est fonction de la pression partielle du diester de 1,4-butanediol injecté dans le four de pyrolyse. Il est typiquement de 1 seconde pour une pression partielle de diester de diol de 0,1 MPa, et de 7 secondes pour une pression partielle de diester de diol de 0,04 MPa. Le temps de contact est défini comme le rapport du volume réactionnel du réacteur sur le débit volumique de charge, le débit volumique étant calculé dans les conditions de température et de pression du milieu réactionnel.

Conformément à l'invention, l'effluent issu dudit réacteur de pyrolyse est refroidi rapidement à une température inférieure à 100°C, de préférence inférieure à 50°C, de manière à limiter la formation de produits de dégradation par exemple par réaction de Diels-Alder de 1,3-butadiène sur lui-même pour former du VinylCycloHexène (VCH). Le refroidissement de l'effluent génère une phase liquide et une phase vapeur qui peuvent être aisément séparées au sein d'un ballon séparateur gaz-liquide en un effluent de pyrolyse liquide et un effluent de pyrolyse vapeur.

Ledit effluent de pyrolyse vapeur comprend plus de 90% poids, de préférence plus de 95% poids de 1,3-butadiène (sans considérer l'éventuel diluant inerte utilisé pour abaisser la pression partielle de diester de 1,4-butanediol au sein du four de pyrolyse). Ledit effluent de pyrolyse vapeur peut également contenir des composés organiques légers, issus de la pyrolyse de l'acide carboxylique, comme par exemple dans le cas où l'acide carboxylique est de l'acide acétique, du méthane, du monoxyde de carbone, du dioxyde de carbone, du cétène, de l'hydrogène ou encore de l'éthane. Ledit effluent de pyrolyse vapeur peut être comprimé et/ou refroidi de manière à condenser le 1,3-butadiène en un effluent 1,3-butadiène. Les composés organiques non condensables issus de la pyrolyse de l'acide carboxylique sont ainsi éliminés en tête d'un séparateur gaz-liquide sous forme d'un effluent de composés légers. Ledit effluent 1,3-butadiène peut ensuite subir une ou plusieurs étapes de purifications ultimes bien connues de l'homme du métier. On peut citer de manière non limitative la purification sur tamis ou sur une argile, un lavage à l'eau suivit d'une étape de séchage. Ceci permet d'éliminer les dernières traces d'impuretés et d'obtenir un effluent 1,3-butadiène, qui comprend plus de 99%, de manière préférée plus de 99,5% de 1,3-butadiène.

Ledit effluent de pyrolyse liquide est composé majoritairement d'acide carboxylique. Par majoritairement, on entend au moins 50% poids, et de préférence au moins 70% poids. Il peut également comprendre d'autres composés organiques, comme par exemple du diester de butanediol non converti, des intermédiaires de pyrolyse (c'est-à-dire des molécules de diester de 1,4-butanediol ayant perdu un fragment acide carboxylique sur les deux nécessaires à la formation du 1,3-butadiène), et d'éventuels sous-produits.

Dans le procédé selon l'art antérieur où une charge 2,3-butanediol et l'acide acétique sont utilisés, de nombreux intermédiaires de pyrolyse et impuretés sont produits. Dans le cas où l'acide acétique est utilisé pour réaliser l'estérification du 2,3-butanediol, le liquide pyrolytique comprend du diacétate de 2,3-butanediol (1,5%pds.), des intermédiaires de pyrolyse tels que, le méthylvinylcarbinol acétate (MVCA 0,8%pds.), le méthyléthylcétoneénol acétate (MEKEA 2,4%pds.) et le crotylacétate (CA 3,3%pds.) et des sous-produits tels que le VCH (2,2%pds), la MEK (1,4%pds.) ou la méthylacétylacétone (MAA 0.9%pds.). Ces intermédiaires ont une température d'ébullition proche de celle de l'acide acétique, et forment des azéotropes homogènes (maximum et minimum) avec ce dernier. La purification de l'acide acétique en vue de son recyclage à l'étape d'estérification ne peut donc se faire par simple distillation. Il est nécessaire de mettre en oeuvre une distillation azéotropique hétérogène, en utilisant l'eau comme entraineur. Outre le fait que cette opération est complexe, elle produit un flux d'acide acétique riche en eau, qui ne peut être directement renvoyé vers l'étape d'estérification. Ce flux d'acide acétique riche en eau doit donc être renvoyé vers une seconde distillation azéotropique hétérogène, afin d'éliminer l'eau. Par ailleurs, cette opération produit une huile qui contient le diacétate 2,3-butanediol non converti, les intermédiaires de pyrolyse, et les sous-produits. La récupération des produits valorisables au sein de cette huile est là aussi problématique car, entre autre, le diacétate 2,3-butanediol non converti et la MAA sont très difficilement séparable par simple distillation.

De manière surprenante, la pyrolyse du diester de 1,4-butanediol conduit à un nombre beaucoup plus restreint d'intermédiaires de pyrolyse et sous-produits, compte tenu de l'amélioration de la conversion et de la sélectivité constatée. Dans le cas préféré où l'acide acétique est utilisé pour réaliser l'estérification du 1,4-butanediol, le liquide pyrolytique comprend comme unique intermédiaire de pyrolyse l'acétate de 3-butèn-1-ol à moins de 4% pds., voire moins de 1% pds., et comme sous-produits des traces de VCH et de THF.

L'effluent de pyrolyse liquide obtenu par le procédé selon l'invention ne contient qu'un seul intermédiaire de pyrolyse ayant une température d'ébullition proche de celle de l'acide carboxylique, et formant un azéotrope homogène avec ce dernier. Cet azéotrope est riche en ester de 3-butèn-1-ol.

De manière avantageuse, ledit effluent de pyrolyse liquide est purifié de manière à produire un flux d'acide carboxylique comprenant l'acide carboxylique débarrassé des impuretés.

Dans un mode de réalisation de l'invention, la purification de l'acide carboxylique peut être réalisée par distillation simple de l'effluent de pyrolyse liquide, produisant un flux d'acide carboxylique, et un flux d'ester de 3-butèn-1-ol (azéotrope acide carboxylique / ester de 3-butèn-1-ol) pouvant être recyclé à l'étape de pyrolyse avec le diester de 1,4-butanediol, ou pouvant subir un traitement pyrolytique dédié.

Dans un autre mode de réalisation de l'invention, la purification de l'acide carboxylique peut être réalisée par distillation avec changement de pression. L'augmentation de pression permet de faire disparaître l'azéotrope, et d'accroître la différence de température d'ébullition entre l'acide carboxylique et l'ester de 3-butèn-1-ol. On obtient ainsi d'une part un flux d'acide carboxylique, et d'autre part un flux d'ester de 3-butèn-1-ol pur pouvant être recyclé à l'étape de pyrolyse avec le diester de 1,4-butanediol, ou pouvant subir un traitement pyrolytique dédié.

Avantageusement, ledit flux comprenant majoritairement de l'acide carboxylique alimentant la section de réaction de ladite étape a) est constitué dudit effluent acide carboxylique issu de la section de séparation de ladite étape a) et dudit flux d'acide carboxylique produit par l'un ou l'autre de ces modes de réalisation (purification de l'acide carboxylique réalisée par distillation simple ou réalisé par distillation avec changement de pression).

La purification de l'effluent de pyrolyse liquide en vue de son recyclage vers l'étape a) est donc grandement simplifiée dans le procédé selon l'invention en comparaison du procédé selon l'art antérieur.

Dans un autre mode de réalisation de l'invention, l'effluent de pyrolyse liquide est directement renvoyé à l'étape a) compte tenu de sa grande pureté en acide carboxylique, sans étape de purification de l'acide carboxylique.

### Etape c) optionnelle de conversion du THF

Dans un arrangement préféré, le procédé selon l'invention comprend également une étape c) de conversion du THF alimentée par ledit effluent THF avantageusement produit dans la section de séparation de l'étape a), et un effluent anhydride d'acide, opérée en présence d'un catalyseur acide, dans laquelle le THF est converti en diester de 1,4-butanediol par acétolyse.

En effet, l'effluent THF avantageusement récupéré au sein de ladite section de séparation de ladite étape a) peut être transformé en diester de 1,4-butanediol en le faisant réagir avec de l'anhydride d'acide en présence d'un catalyseur acide. L'anhydride d'acide mis en oeuvre dans cette étape c) de conversion du THF correspond avantageusement à l'acide carboxylique utilisé à l'étape a) pour réaliser l'estérification du 1,4-butanediol. Par exemple, dans le mode préféré de l'invention où de l'acide acétique est utilisé pour transformer le 1,4-butanediol en diacétate de 1,4-butanediol à l'étape a) de conversion, on utilisera avantageusement l'anhydride acétique à l'étape c) de conversion du THF. Cette réaction de conversion du THF peut être réalisée avec ou sans solvant. Dans un mode préféré de l'invention, le solvant utilisé est l'acide carboxylique utilisé à l'étape a) de conversion, à condition que ce dernier ait été préalablement séché, car l'eau réagirait avec l'anhydride d'acide.

Cette étape c) de conversion du THF peut être réalisée de manière continue ou, si la quantité de THF produite est faible, de manière séquentielle (mise en oeuvre « batch », selon la terminologie anglo-saxonne).

Cette étape c) optionnelle de l'invention permet, via une faible consommation d'anhydride d'acide, de ne pas avoir de sous-produit THF à éliminer du procédé et d'augmenter le rendement global du procédé en 1,3-butadiène, contrairement au procédé de l'art antérieur qui produit de la MEK en grande quantité, laquelle ne peut être valorisée en 1,3-butadiène.

Par ailleurs, l'ajout d'anhydride d'acide permet de réaliser l'appoint d'acide carboxylique nécessaire pour compenser les pertes en acide carboxylique à l'étape de pyrolyse b). En effet, d'un point de vue purement stoechiométrique, une mole de THF et une mole d'anhydride d'acide donnent une mole de diester de 1,4-butanediol à l'étape c) de conversion du THF. Cette mole de diester de 1,4-butanediol est ensuite envoyée, directement ou indirectement à une étape de pyrolyse, où d'un point de vue purement stoechiométrique une mole de diester de 1,4-butanediol donne une mole de 1,3-butadiène et deux moles d'acide carboxylique. Dans la pratique, l'anhydride d'acide est introduit en excès par rapport au THF, de manière à garantir une conversion quasi-totale de ce dernier et tenir compte du fait que l'effluent THF issu de la section de séparation de l'étape a) n'est pas parfaitement anhydre. Les traces d'eau dans le THF vont donc consommer une partie de l'anhydride d'acide introduit à l'étape c), pour produire deux moles d'acide carboxylique.

La conversion du THF en diacétate de 1,4-butanediol est décrite, par exemple, dans Tetrahedron Letters, 45(35), p.6599-6602 et dans Journal of Heterocylclic Chemistry, 37(5), pages 1351-1353. Le THF est converti en diacétate de 1,4-butanediol dans l'acide acétique, en présence d'un catalyseur acide et d'anhydride acétique, le catalyseur acide pouvant être par exemple de l'acide sulfamique ou de l'acide sulfurique, à une température comprise entre 20°C et 80°C, pendant une durée allant de 2h à 25h. Des rendements de conversion de plus de 97%mol sont atteints.

Dans un mode de réalisation de l'invention, l'effluent issu de l'étape c) de conversion du THF en diester de 1,4-butanediol est envoyé directement à l'étape b) de pyrolyse du diester de 1,4-butanediol ou bien subit une étape de pyrolyse dédiée, dans le but de produire d'avantage de butadiène.

Dans un autre mode de réalisation de l'invention, l'effluent issu de l'étape c) de conversion du THF en diester de 1,4-butanediol est renvoyé à l'étape a) d'estérification de la charge 1,4-butanediol. Cet effluent est avantageusement introduit dans la partie inférieure de ladite colonne de distillation réactive, éventuellement avec l'acide carboxylique.

L'effluent issu de l'étape c) contient du diester de 1,4-butanediol, de l'anhydride acide non consommé, éventuellement de l'acide carboxylique utilisé comme solvant ou produit par hydrolyse de l'anhydride acide, une faible proportion de THF non converti et est exempt de la moindre trace d'eau. L'effluent issu de l'étape c) peut donc être renvoyé dans ladite section réactionnelle de ladite étape a), avantageusement dans ladite colonne de distillation réactive, sans en dégrader les performances, et permet même de les améliorer.

En effet, le THF et l'acide carboxylique sont les corps les plus légers et migrent vers la tête de ladite colonne de distillation réactive, alors que l'anhydride d'acide et le diester de 1,4-butanediol sont les corps les plus lourds et migrent vers le fond de ladite colonne de distillation réactive. Ainsi, l'anhydride d'acide non consommé à l'étape c) de conversion du THF réagit avec la très faible fraction de monoester de 1,4-butanediol et les traces de 1,4-butanediol non converties en fond de ladite colonne de distillation réactive permettant, toute choses égales par ailleurs, d'améliorer la conversion de la charge 1,4-butanediol en diester de 1,4-butanediol à l'étape a).

Par ailleurs, suivant la quantité d'anhydride acétique non consommé à l'étape c) renvoyé à l'étape a), les réglages (c'est à dire taux de rebouillage, taux de reflux, rapport des débits de charge 1,4-butanediol sur débit d'acide acétique,...) de ladite colonne de distillation réactive peuvent être adaptées dans le but de réduire la consommation énergétique de l'étape a). En effet, la variation de la consommation énergique de l'étape a) n'est pas linéaire avec la conversion de la charge 1,4-butanediol, les derniers % de conversion étant plus difficiles à obtenir pour une réaction équilibrée. L'utilisation de l'anhydride acide non converti à l'étape c) pour réaliser les derniers % de conversion de la charge 1,4-butanediol permet donc de réduire fortement la consommation énergétique de l'étape a).

### Description des figures

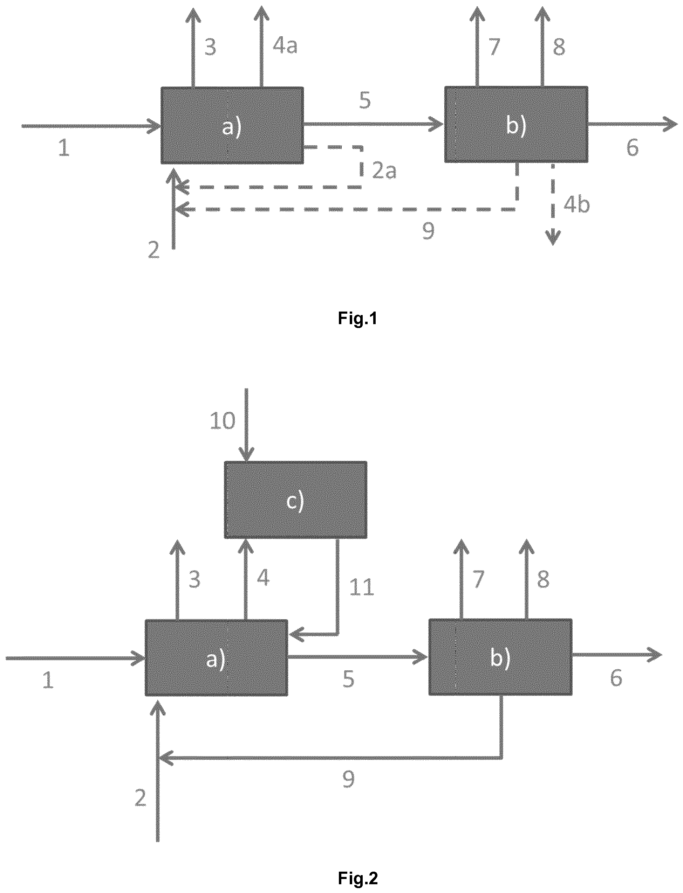

La figure 1 présente une vue schématique d'un arrangement spécifique du procédé selon l'invention.

La charge 1,4-butanediol (1) subit une première étape d'estérification a) avec un flux d'acide carboxylique (9) et un flux comprenant majoritairement de l'acide carboxylique (2), pour produire un effluent eau (3), un effluent diester de 1,4-butanediol (5) et un effluent acide carboxylique (2a). Durant l'étape a), une faible fraction de la charge 1,4-butanediol est convertie en THF qui est éliminée de l'étape a) dans l'effluent THF 4a). L'effluent acide carboxylique (2a) est recyclé en mélange avec le flux comprenant majoritairement de l'acide carboxylique (2).

L'effluent diester de 1,4-butandiol (5) alimente une étape de pyrolyse b) qui produit un effluent 1,3-butadiène (7) et un effluent de pyrolyse liquide (9) qui est recyclé vers l'étape d'estérification a). Durant l'étape b) de pyrolyse, une très faible fraction du diester de 1,4-butanediol est convertie en THF, qui peut éventuellement être éliminée de l'étape b) dans l'effluent d'ester de 3 butèn-1-ol 4b) ou envoyé via le flux (9) vers l'étape a) pour être *in fine* éliminé de l'étape a) par le flux 4a).

Par ailleurs, durant l'étape b) de pyrolyse, une faible fraction de l'acide carboxylique est craquée. Les produits de craquage sont éliminées de l'étape b) dans l'effluent de composés légers (8). L'appoint d'acide carboxylique (2) introduit à l'étape a) compense les pertes en acide carboxylique à l'étape b). Une faible proportion de coke est également produite, qui est évacuée par l'effluent coke (6).

La figure 2 présente un arrangement particulier de l'étape d'estérification avec mise en oeuvre de l'étape c) de conversion du THF du procédé selon l'invention.

L'étape a) d'estérification est alimentée par la charge 1,4-butanediol (1) d'une part et par un flux d'acide carboxylique (9) provenant de l'étape de pyrolyse (b), l'effluent (11) de conversion du THF issu de l'étape c) et un appoint d'acide carboxylique (2) d'autre part. L'étape a) produit un effluent diester de 1,4-butanediol (5) qui est envoyé à l'étape b) de pyrolyse, un effluent THF (4) qui est envoyé à l'étape c) de conversion du THF et un effluent eau (3) qui est éliminé du procédé. L'étape b) de pyrolyse est alimentée par l'effluent diester de 1,4-butanediol (5) issu de l'étape a) et produit un effluent 1,3-butadiène (7), un effluent de composés légers (8) contenant les produits de craquage de l'acide carboxylique, et un flux d'acide carboxylique (9) qui est renvoyé vers l'étape a). Lors de la pyrolyse, une faible proportion de coke est également produite, qui est évacuée par l'effluent coke (6). L'étape c) est alimentée par l'effluent THF (4) issu de l'étape a), et par l'effluent anhydride acide (10), et produit un effluent de conversion du THF (11) qui est renvoyé vers l'étape a).

### EXEMPLE

### Exemple 1 - Estérification du 2,3-butanediol (comparatif)

*Cet exemple montre les performances d'un procédé d'estérification mis en oeuvre selon l'enseignement de l'art antérieur.*

L'estérification du 2,3-butanediol est réalisée dans un réacteur agité fermé, dans les conditions suivantes :
- un température de 110°C (correspond à la température moyenne de la zone réactive de la colonne à distiller réactive a1)).
- 6 moles d'acide acétique par mole de 2,3-butanediol (correspondant à un large excès d'acide acétique).
- Catalyseur acide TA801 à 2.2%mol. par rapport au 2,3-butanediol.

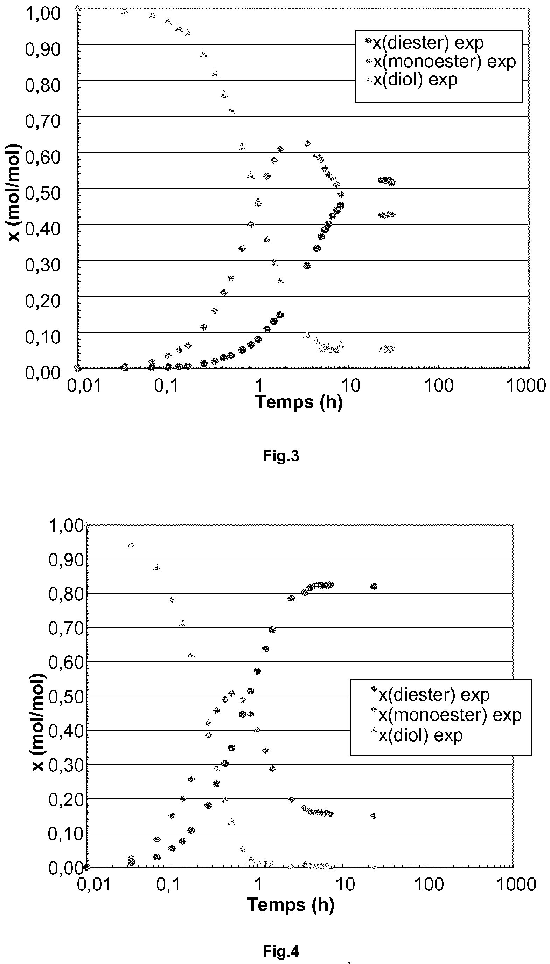

La conversion du 2,3-butanediol en monoester et en diester au cours du temps est suivie par chromatographie gazeuse (Figure 3). A noter que la cinétique de conversion des formes RR, SS et RS du 2,3-butanediol sont identiques et la proportion des différents diastéréoisomères a été sommée. On peut constater que malgré la présence d'un large excès d'acide acétique, la conversion du 2,3-butanediol est limitée, ce qui justifie la mise en oeuvre d'une distillation réactive de manière à déplacer l'équilibre vers la formation du diester de 2,3-butanediol.

Néanmoins, on peut constater que la cinétique d'estérification est plutôt lente malgré la présence du catalyseur acide, étant donné qu'il faut plus de 20 heures pour atteindre l'équilibre thermodynamique.

### Exemple 2 - Estérification du 1,4-butanediol (invention)

*Cet exemple montre les performances d'un procédé d'estérification mis en oeuvre selon l'invention.*

L'estérification du 1,4-butanediol est réalisée dans un réacteur agité fermé, dans les conditions suivantes :
- un température de 110°C (correspond à la température moyenne de la zone réactive de la colonne à distiller réactive a1)).
- 6 moles d'acide acétique par mole de 1,4-butanediol (correspondant à un large excès d'acide acétique).
- Catalyseur acide TA801 à 2.2%mol. par rapport au 1,4-butanediol.

La conversion du 1,4-butanediol en monoester et en diester au cours du temps est suivie par chromatographie gazeuse (Figure 4). On peut constater que, toute choses égales par ailleurs, la conversion du 1,4-butanediol est bien supérieure à celle 2,3-butanediol. Industriellement, cela va se traduire par une réduction du rapport acide acétique / butanediol, et donc par une réduction des coûts d'opération et d'investissement associés à l'étape d'estérification.

Par ailleurs, on peut constater que la cinétique d'estérification du 1,4-butanediol est plus rapide que celle du 2,3-butanediol, puisque l'équilibre thermodynamique est atteint en 3h dans le cas de l'estérification du 1,4-butanediol, contre 20 heures dans le cas de l'estérification du 2,3-butanediol. Industriellement, cela va se traduire par une réduction du temps de séjour au sein de la colonne de distillation réactive, et donc par une réduction des coûts d'investissement associés à cette opération.

### Exemple 3 - Pyrolyse du diacétate de 2,3-butanediol (comparatif)

*Cet exemple montre les performances d'un procédé de pyrolyse mis en oeuvre selon l'enseignement de l'art antérieur.*

Une charge composée de diacetate de 2,3-butanediol alimente un four de pyrolyse. La température du four de pyrolyse est réglée de façon à obtenir une température en sortie de réacteur de 630°C. Le débit de diacétate de 2,3-butanediol est réglé pour obtenir un temps de séjour entre 0.9 et 1.5 secondes. L'effluent de pyrolyse est rapidement refroidi à 45°C avec un condenseur placé en sorti immédiate du four de pyrolyse. La conversion de diacetate de 2,3-butanediol varie entre 96.5% et 99.1% en fonction du temps de séjour. La sélectivité de transformation du diacétate de 2,3-butanediol en 1,3-butadiène est comprise entre 78 % et 82%. La sélectivité de transformation du diacétate de 2,3-butanediol en intermédiaires de pyrolyse varie entre 17.2% et 20.0%. La sélectivité de transformation du diacétate de 2,3-butanediol en MEK, varie entre 0.9% et 1.8%. Il n'y a aucun autre produit de conversion détecté.

Parmi les intermédiaires de pyrolyse, certains permettent d'augmenter le rendement global en dioléfine du procédé s'ils sont recyclés à l'étape de pyrolyse, et d'autres non. Le méthyl vinyl carbinol acétate (MVCA) et le crotyl acétate (CA) permettent d'augmenter le rendement en butadiène s'ils sont recyclés à l'étape de pyrolyse, alors que ce n'est pas le cas du méthyl éthyl cétone énol acétate (MEKEA).

Or, ces intermédiaires de pyrolyse sont des isomères, et ont donc des propriétés physico-chimiques très proches. Par ailleurs, ces intermédiaires de pyrolyse sont fortement dilués dans l'acide carboxylique. Il s'avère que lorsque la charge diol est un butanediol, quel que soit l'acide carboxylique considéré, la volatilité relative entre l'acide carboxylique et les intermédiaires de pyrolyse est très proche de l'unité. L'ensemble de ces éléments rend l'extraction des intermédiaires de pyrolyse au sein de l'effluent liquide de pyrolyse, dans le but de maximiser le rendement, très difficile.

### Exemple 4 - Pyrolyse du diacétate de 1,4-butanediol (invention)

*Cet exemple montre les performances d'un procédé de pyrolyse mis en oeuvre selon l'invention.*

Une charge composée de diacétate de 1,4-butanediol alimente un four de pyrolyse. La température du four de pyrolyse est réglée de façon à obtenir une température en sortie de réacteur de 630°C. Le débit de diacetate de 1,4-butanediol est réglé pour obtenir un temps de séjour entre 1 et 2 secondes. L'effluent de pyrolyse est rapidement refroidi à 45°C avec un condenseur placé en sorti immédiate du four de pyrolyse. La conversion de diacétate de 1,4-butanediol varie entre 96.2% et 100%, en fonction du temps de séjour. La sélectivité de transformation du diacétate de 1,4-butanediol en 1,3-butadiène est comprise entre 96,3% et 100%.La sélectivité de transformation du diacétate de 1,4-butanediol en 3-butèn-1-ol (seul intermédiaire de pyrolyse) varie entre 0% et 3.7%. On n'observe pas de formation de THF. Aucun autre produit n'est détecté.

Par cet exemple nous démontrons que l'utilisation du 1,4-butanediol comme charge d'un procédé d'estérification et pyrolyse selon l'invention pour la production du 1,3-butadiène présente l'avantage d'avoir un rendement molaire supérieur en butadiène et de former moins de produits secondaires..

### Exemple 5 - Production de butadiène à partir d'une charge 1,4-butanediol (invention)

*Cet exemple illustre la production de 1,3-butadiène à partir d'une charge 1,4-butanediol par le procédé selon l'invention, dans sa variante avec (variante 5-a) et sans (variante 5-b) mise en oeuvre de l'étape c).*

### Variante 5-a (invention), sans mise en oeuvre de l'étape c) de conversion du THF

Le tableau ci-dessous donne les débits et la composition des différents flux au sein du procédé selon l'invention, la numérotation des flux étant identique à celui de la Figure 1.

La charge 1,4-butanediol (1) contenant principalement du 1,4-butanediol, mais aussi 3,7%pds d'eau, alimente une étape a) d'estérification. Cette étape a) d'estérification est par ailleurs alimentée par le flux (9) issu de l'étape b) de pyrolyse et qui est constitué principalement d'acide acétique, et par le flux (2) d'acide acétique pur qui permet de compenser les pertes d'acide acétique au sein du procédé. Cette étape a) d'estérification produit par ailleurs le flux (3) d'eau qui est éliminé du procédé, le flux (4a) de THF qui est éliminé du procédé, et le flux (5) de diester de 1,4-butanediol qui est envoyé à l'étape b) de pyrolyse.

L'étape b) de pyrolyse produit flux (7) de 1,3-butadiène, un flux (8) de produits incondensables issus de la pyrolyse de l'acide acétique, et un flux (9) constitué principalement d'acide acétique et qui est renvoyé à l'étape a) d'estérification.

Du coke est aussi produit lors de la pyrolyse, qui est éliminé par le flux (6) lors de la régénération du four de pyrolyse.

| Flux | (1) | (2) | (3) | (4a) | (5) | (6) | (7) | (8) | (9) |
|---|---|---|---|---|---|---|---|---|---|
| Débit (kg/h) | 9358 | 572 | 3907 | 158 | 17250 | 344 | 5186 | 335 | 11384 |

| Composition (%pds) | | | | | | | | | |
|---|---|---|---|---|---|---|---|---|---|
| 1,4-Butanediol | 96,3 | - | - | - | - | - | - | - | - |
| Eau | 3,7 | - | 100 | - | - | - | - | - | - |
| Acide Acétique | - | 100 | - | - | 0,14 | - | - | - | 98,94 |
| THF | - | - | - | 100 | - | - | - | - | 0,06 |
| Diester | - | - | - | - | 99,78 | - | - | - | - |
| Monoester | - | - | - | - | 0,08 | - | - | - | - |
| 1,3-Butadiène | - | - | - | - | - | - | 100 | - | - |
| Intermédiaire | - | - | - | - | - | - | - | - | 1,00 |
| incondensables | - | - | - | - | - | - | - | 100 | - |
| coke | - | - | - | - | - | 100 | - | - | - |

Ainsi 0,57 tonnes de butadiène par tonnes de 1,4-butanediol sont produits par le procédé selon l'invention, soit un rendement de 95,9% du rendement stoechiométrique. La perte de rendement provient de différentes réactions indésirables :
- la production de THF par déshydratation du 1,4-butanediol à l'étape a) d'estérification,
- la production de THF par hydrolyse de l'intermédiaire de pyrolyse, lorsque ce dernier n'est pas isolé mais directement recyclé vers l'étape a) d'estérification,
- le production de THF par pyrolyse du monoester de 1,4-butanediol à l'étape b) de pyrolyse,
- et enfin la production de coke.

### Variante 5-b (invention), avec mise en oeuvre de l'étape c) de conversion du THF

Le tableau ci-dessous donne les débits et la composition des différents flux au sein du procédé selon l'invention, avec mise en oeuvre de l'étape c) de conversion du THF. Le numéro des flux étant identique à celui de la Figure 2.

La charge 1,4-butanediol (1) contenant principalement du 1,4-butanediol, mais aussi 3,7%pds d'eau, alimente une étape a) d'estérification. Cette étape a) d'estérification est par ailleurs alimentée par le flux (9) issu de l'étape b) de pyrolyse et qui est constitué principalement d'acide acétique, et par le flux (2) d'acide acétique pur qui permet de compenser les pertes d'acide acétique au sein du procédé. Cette étape a) d'estérification produit par ailleurs le flux (3) d'eau qui est éliminé du procédé, le flux (4) de THF qui est envoyé à l'étape c) de conversion du THF, et le flux (5) de diester de 1,4-butanediol qui est envoyé à l'étape b) de pyrolyse.

L'étape c) est alimentée par le flux (4) issu de l'étape a) et par le flux (10) d'anhydride acétique, et produit un flux (11) qui est renvoyé à l'étape a) d'estérification.

L'étape b) de pyrolyse produit flux (7) de 1,3-butadiène, un flux (8) de produits incondensables issus de la pyrolyse de l'acide acétique, et un flux (9) constitué principalement d'acide acétique et qui est renvoyé à l'étape a) d'estérification.

Du coke est aussi produit lors de la pyrolyse, qui est éliminé par le flux (6) lors de la régénération du four de pyrolyse.

| Flux | (1) | (2) | (3) | (4) | (5) | (6) | (7) | (8) | (9) | (10) | (11) |
|---|---|---|---|---|---|---|---|---|---|---|---|
| Débit (kg/h) | 9358 | 285 | 3901 | 155 | 17623 | 352 | 5302 | 342 | 11626 | 255 | 410 |

| Composition (%pds) | | | | | | | | | | | |
|---|---|---|---|---|---|---|---|---|---|---|---|
| 1,4-Butanediol | 96,3 | - | - | - | - | - | - | - | - | - | - |
| Eau | 3,7 | - | 100 | - | - | - | - | - | - | - | - |
| Acide Acétique | - | 100 | - | - | 0,14 | - | - | - | 99 | - | - |
| Anhydride Ac. | - | - | - | - | traces | - | - | - | - | 100 | 9,7 |
| THF | - | - | - | 100 | - | - | - | - | - | - | 0,7 |
| Diester | - | - | - | - | 99,86 | - | - | - | - | - | 89,6 |
| Monoester | - | - | - | - | traces | - | - | - | - | - | - |
| 1,3-Butadiène | - | - | - | - | - | - | 100 | - | - | - | - |
| Intermédiaire | - | - | - | - | - | - | - | - | 1 | - | - |
| incondensables | - | - | - | - | - | - | - | 100 | - | - | - |
| coke | - | - | - | - | - | 100 | - | - | - | - | - |

Ainsi 0,59 tonnes de butadiène par tonnes de 1,4-butanediol sont produits par le procédé selon l'invention, avec une étape de conversion du THF, soit un rendement de 98% du rendement stoechiométrique. On pourra par ailleurs noter que en comparaison avec l'exemple A, les performances de l'étape a) d'estérification ont été améliorées, de par le recyclage à l'étape a) du flux (11) contenant encore de l'anhydride acétique non converti à l'étape c. Par ailleurs, comme il n'y a plus de monoester de 1,4-butanediol dans le flux (5) qui est envoyé à l'étape b), et il n'y a donc plus de formation de THF à l'étape c) de pyrolyse.

## Revendications

1. Procédé de conversion alimenté par une charge 1,4-butanediol, ledit procédé comprenant au moins :
a) une étape d'estérification comprenant une section de réaction alimentée par la charge 1,4-butendiol et un flux comprenant majoritairement de l'acide carboxylique, comprenant également au moins une section de séparation séparant l'effluent de la section de réaction en au moins un effluent diester de 1,4-butanediol, un effluent eau, et un effluent acide carboxylique, ladite section de réaction étant mise en oeuvre en présence d'un catalyseur acide, à une pression comprise entre 0,01 et 1,0 MPa et à un débit molaire de diol alimentant ladite section sur le nombre de mole de catalyseur dans ladite section compris entre 0,05 et 25 h⁻¹ ;
b) une étape de pyrolyse comprenant un réacteur de pyrolyse alimenté au moins par ledit effluent diester de 1,4-butanediol issu de l'étape a) d'estérification de manière à produire un effluent de pyrolyse, ladite étape de pyrolyse comprenant également au moins une section de séparation dans laquelle ledit effluent de pyrolyse est refroidi à une température inférieure à 100°C de manière à produire au moins un effluent de pyrolyse liquide et un effluent de pyrolyse vapeur, ledit effluent de pyrolyse vapeur étant comprimé et/ou refroidi de manière à condenser le 1,3-butadiène en un effluent 1,3-butadiène.

2. Procédé selon la revendication 1 dans lequel le flux comprenant majoritairement de l'acide carboxylique alimentant la section de réaction de ladite étape a) comprend l'effluent acide carboxylique issu de la section de séparation de l'étape a).

3. Procédé selon l'une des revendications 1 ou 2 dans lequel le flux comprenant majoritairement de l'acide carboxylique alimentant la section de réaction de ladite étape a) comprend un appoint d'acide carboxylique.

4. Procédé selon l'une des revendications 1 à 3 dans lequel ladite section de réaction de ladite étape a) est mise en oeuvre dans une colonne de distillation réactive, dans laquelle la charge 1,4-butanediol est introduite dans la partie supérieure de la colonne, et l'acide carboxylique est introduit dans la partie inférieure de la colonne, le rapport des débits molaires de 1,4-butanediol et d'acide carboxylique étant compris entre 2 et 6.

5. Procédé selon l'une des revendications 1 à 4 dans lequel l'acide carboxylique utilisé est choisi parmi l'acide formique, l'acide acétique, l'acide propanoïque, l'acide butanoïque ou l'acide benzoïque.

6. Procédé selon la revendication 5 dans lequel l'acide carboxylique utilisé est l'acide acétique.

7. Procédé selon la revendication 6 dans lequel ladite section de séparation de ladite étape a) est réalisée par distillation azéotropique hétérogène, en utilisant un entraineur.

8. Procédé selon l'une des revendications 1 à 7 dans lequel ledit flux comprenant majoritairement de l'acide carboxylique alimentant la section de réaction de ladite étape a) comprend ledit effluent de pyrolyse liquide.

9. Procédé selon l'une des revendications 1 à 7 dans lequel ledit effluent de pyrolyse liquide est séparé par distillation simple de manière à produire un flux d'acide carboxylique, et un flux d'ester de 3-butèn-1-ol (azéotrope acide carboxylique / ester de 3-butèn-1-ol).

10. Procédé selon l'une des revendications 1 à 7 dans lequel l'effluent de pyrolyse liquide est séparé par distillation avec changement de pression de manière à produire un flux d'acide carboxylique, et d'autre part un flux d'ester de 3-butèn-1-ol.

11. Procédé selon l'une des revendications 9 ou 10 dans lequel ledit flux comprenant majoritairement de l'acide carboxylique alimentant la section de réaction de ladite étape a) comprend ledit flux d'acide carboxylique issu de l'effluent de pyrolyse liquide.

12. Procédé selon l'une des revendications 9 ou 10 dans lequel ledit flux d'ester de 3-butèn-1-ol est recyclé à l'étape b) de pyrolyse avec le diester de 1,4-butanediol.

13. Procédé selon l'une des revendications 1 à 12 dans lequel un effluent THF est séparé de l'effluent de ladite section de réaction de ladite étape a).

14. Procédé selon la revendication 13 comprenant également une étape c) de conversion du THF alimentée par ledit effluent THF et un effluent anhydride d'acide, opérée en présence d'un catalyseur acide.

15. Procédé selon la revendication 14 dans lequel l'effluent issu de l'étape c) de conversion du THF en diester de 1,4-butanediol est envoyé directement à l'étape b) de pyrolyse du diester de 1,4-butanediol ou bien subit une étape de pyrolyse dédiée, dans le but de produire d'avantage de butadiène.

16. Procédé selon la revendication 14 dans lequel l'effluent issu de l'étape c) de conversion du THF en diester de 1,4-butanediol est renvoyé à l'étape a) d'estérification de la charge 1,4-butanediol.

## Patentansprüche

1. Umwandlungsverfahren, das mit einer 1,4-Butandiol-Charge versorgt wird, wobei das Verfahren mindestens umfasst:
a) einen Veresterungsschritt, umfassend einen Reaktionsabschnitt, der mit der 1,4-Butandiol-Charge und einem Strom, umfassend mehrheitlich Carbonsäure, versorgt wird, auch umfassend mindestens einen Trennungsabschnitt, der den Reaktionsabfluss in mindestens einen 1,4-Butandiol-Diester-Abfluss, einen Wasserabfluss und einen Carbonsäureabfluss trennt, wobei der Reaktionsabschnitt im Beisein eines sauren Katalysators bei einem Druck zwischen 0,01 und 1,0 MPa und einer Molmenge von Diol, die den Abschnitt versorgt, zur Katalysatormolzahl in dem Abschnitt zwischen 0,05 und 25 h⁻¹ eingesetzt wird;
b) einen Pyrolyseschritt, umfassend einen Pyrolysereaktor, der zumindest mit dem 1,4-Butandiol-Diester-Abfluss aus dem Veresterungsschritt a) versorgt wird, um einen Pyrolyseabfluss zu erzeugen, wobei der Pyrolyseschritt auch mindestens einen Trennungsabschnitt umfasst, in dem der Pyrolyseabfluss auf eine Temperatur unter 100 °C gekühlt wird, um mindestens einen flüssigen Pyrolyseabfluss und einen dampfförmigen Pyrolyseabfluss zu erzeugen, wobei der dampfförmige Pyrolyseabfluss komprimiert und/oder gekühlt wird, um das 1,3-Butandien zu einem 1,3-Butandien-Abfluss zu kondensieren.

2. Verfahren nach Anspruch 1, bei dem der Strom, mehrheitlich umfassend Carbonsäure, die den Reaktionsabschnitt des Schritts a) versorgt, den Carbonsäureabfluss aus dem Trennungsabschnitt des Schritts a) umfasst.

3. Verfahren nach einem der Ansprüche 1 oder 2, bei dem der Strom, umfassend mehrheitlich Carbonsäure, die den Reaktionsabschnitt des Schritts a) versorgt, einen Zusatz von Carbonsäure umfasst.

4. Verfahren nach einem der Ansprüche 1 bis 3, bei dem der Reaktionsabschnitt des Schritts a) in einer reaktiven Destillationskolonne eingesetzt wird, in die die 1,4-Butandiol-Charge in den oberen Teil der Kolonne eingeführt wird, und die Carbonsäure in den unteren Teil der Kolonne eingeführt wird, wobei das Verhältnis der Molmengen von 1,4-Butandiol und Carbonsäure zwischen 2 und 6 liegt.

5. Verfahren nach einem der Ansprüche 1 bis 4, bei dem die verwendete Carbonsäure unter Ameisensäure, Essigsäure, Propansäure, Butansäure oder Benzoesäure ausgewählt ist.

6. Verfahren nach Anspruch 5, bei dem die verwendete Carbonsäure Essigsäure ist.

7. Verfahren nach Anspruch 6, bei dem der Trennungsabschnitt des Schritts a) durch heterogene azeotrope Destillation unter Verwendung eines Schleppmittels durchgeführt wird.

8. Verfahren nach einem der Ansprüche 1 bis 7, bei dem der Strom, umfassend mehrheitlich Carbonsäure, die den Reaktionsabschnitt des Schritts a) versorgt, den flüssigen Pyrolyse-Abfluss umfasst.

9. Verfahren nach einem der Ansprüche 1 bis 7, bei dem der flüssige Pyrolyse-Abfluss durch einfache Destillation getrennt wird, um einen Carbonsäurestrom und einen Strom von 3-Butenester-1-ol (azeotrope Carbonsäure/3-Butenester-1-ol) zu erzeugen.

10. Verfahren nach einem der Ansprüche 1 bis 7, bei dem der flüssige Pyrolyse-Abfluss durch Destillation mit Druckänderung getrennt wird, um einen Carbonsäurestrom und andererseits einen Strom von 3-Butenester-1-ol zu erzeugen.

11. Verfahren nach einem der Ansprüche 9 oder 10, bei dem der Strom, umfassend mehrheitlich Carbonsäure, die den Reaktionsabschnitt des Schritts a) versorgt, den Carbonsäurestrom umfasst, der von dem flüssigen Pyrolyse-Abfluss stammt.

12. Verfahren nach einem der Ansprüche 9 oder 10, bei dem der Strom von 3-Butenester-1-ol in dem Pyrolyseschritt b) mit dem 1,4-Butandiol-Diester wiederverwertet wird.

13. Verfahren nach einem der Ansprüche 1 bis 12, bei dem ein THF-Abfluss von dem Abfluss des Reaktionsabschnitts des Schritts a) getrennt wird.

14. Verfahren nach Anspruch 13, auch umfassend einen Schritt c) der Umwandlung des THF, das mit dem THF-Abfluss und einem wasserfreien Säureabfluss versorgt wird, der im Beisein eines sauren Katalysators durchgeführt wird.

15. Verfahren nach Anspruch 14, bei dem der Abfluss aus dem Schritt c) der Umwandlung des THF in 1,4-Butandiol-Diester direkt in den Schritt b) der Pyrolyse des 1,4-Butandiol-Diesters geschickt wird oder auch einem speziellen Pyrolyseschritt unterzogen wird, um mehr Butadien zu erzeugen.

16. Verfahren nach Anspruch 14, bei dem der Abfluss aus dem Schritt c) der Umwandlung des THF in 1,4-Butandiol-Diester in den Schritt a) der Veresterung der 1,4-Butandiol-Charge zurückgeschickt wird.

## Claims

1. Method for conversion fed with a 1,4-butanediol feedstock, wherein said method comprises at least:
a) An esterification step that comprises a reaction section that is fed with the 1,4-butenediol feedstock and a flow that comprises for the most part carboxylic acid, also comprising at least one separation section that separates the effluent from the reaction section into at least one 1,4-butanediol diester effluent, a water effluent, and a carboxylic acid effluent, with said reaction section being implemented in the presence of an acid catalyst at a pressure of between 0.01 and 1.0 MPa and at a molar flow rate of diol feeding said section with a catalyst mol number in said section of between 0.05 and 25 h⁻¹;
b) A step for pyrolysis comprising a pyrolysis reactor that is fed with at least said 1,4-butanediol diester effluent that is obtained from the esterification step a) in such a way as to produce a pyrolysis effluent, with said step for pyrolysis also comprising at least one separation section in which said pyrolysis effluent is cooled to a temperature that is less than 100°C in such a way as to produce at least one liquid pyrolysis effluent and one vapor pyrolysis effluent, with said vapor pyrolysis effluent being compressed and/or cooled in such a way as to condense 1,3-butadiene into a 1,3-butadiene effluent.

2. Method according to Claim 1, wherein the flow that comprises for the most part carboxylic acid that feeds the reaction section of said step a) comprises the carboxylic acid effluent that is obtained from the separation section of step a).

3. Method according to one of Claims 1 or 2, wherein the flow that comprises for the most part carboxylic acid that feeds the reaction section of said step a) comprises an addition of carboxylic acid.

4. Method according to one of Claims 1 to 3, wherein said reaction section of said step a) is implemented in a reactive distillation column, in which the 1,4-butanediol feedstock is introduced into the upper part of the column, and the carboxylic acid is introduced into the lower part of the column, with the ratio of the molar flow rates of 1,4-butanediol and carboxylic acid being between 2 and 6.

5. Method according to one of Claims 1 to 4, wherein the carboxylic acid that is used is selected from among formic acid, acetic acid, propanoic acid, butanoic acid, or benzoic acid.

6. Method according to Claim 5, wherein the carboxylic acid that is used is acetic acid.

7. Method according to Claim 6, wherein said separation section of said step a) is produced by heterogeneous azeotropic distillation using a driver.

8. Method according to one of Claims 1 to 7, wherein said flow that comprises for the most part carboxylic acid that feeds the reaction section of said step a) comprises said liquid pyrolysis effluent.

9. Method according to one of Claims 1 to 7, wherein said liquid pyrolysis effluent is separated by simple distillation in such a way as to produce a carboxylic acid flow and a 3-buten-1-ol ester flow (carboxylic acid azeotrope/3-buten-1-ol ester).

10. Method according to one of Claims 1 to 7, wherein the liquid pyrolysis effluent is separated by distillation with a change in pressure in such a way as to produce a carboxylic acid flow and, in contrast, a 3-buten-1-ol ester flow.

11. Method according to one of Claims 9 or 10, wherein said flow that comprises for the most part the carboxylic acid that feeds the reaction section of said step a) comprises said carboxylic acid flow that is obtained from the liquid pyrolysis effluent.

12. Method according to one of Claims 9 or 10, wherein said 3-buten-1-ol ester flow is recycled in the pyrolysis step b) with 1,4-butanediol diester.

13. Method according to one of Claims 1 to 12, wherein a THF effluent is separated from the effluent of said reaction section of said step a).

14. Method according to Claim 13 that further comprises a step c) for conversion of THF that is fed with said THF effluent and an acid anhydride effluent, carried out in the presence of an acid catalyst.

15. Method according to Claim 14, wherein the effluent that is obtained from step c) for conversion of THF into 1,4-butanediol diester is sent directly to step b) for pyrolysis of 1,4-butanediol diester or else undergoes a dedicated step for pyrolysis for the purpose of producing more butadiene.

16. Method according to Claim 14, wherein the effluent that is obtained from step c) for conversion of THF into 1,4-butanediol diester is sent back to step a) for esterification of the 1,4-butanediol feedstock.
